(19) Europäisches Patentamt
European Patent Office
Office européen des brevets

(11) EP 3 900 713 A1

(12) **EUROPEAN PATENT APPLICATION**
published in accordance with Art. 153(4) EPC

(43) Date of publication:
**27.10.2021 Bulletin 2021/43**

(21) Application number: **19898804.0**

(22) Date of filing: **19.12.2019**

(51) Int Cl.:
***A61K 31/167*** *(2006.01)* ***A61K 31/196*** *(2006.01)*
***A61P 23/02*** *(2006.01)* ***A61P 29/00*** *(2006.01)*
***A61P 43/00*** *(2006.01)* ***A61K 9/00*** *(2006.01)*
***A61K 9/70*** *(2006.01)* ***A61K 47/10*** *(2017.01)*
***A61K 47/12*** *(2006.01)* ***A61K 47/14*** *(2017.01)*

(86) International application number:
**PCT/JP2019/049856**

(87) International publication number:
**WO 2020/130084 (25.06.2020 Gazette 2020/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.12.2018 JP 2018237691**

(71) Applicant: **MEDRx Co., Ltd.**
**Higashikagawa-shi**
**Kagawa 769-2712 (JP)**

(72) Inventors:
- **KAWAHARA, Haruka**
**Higashikagawa-shi, Kagawa 769-2712 (JP)**
- **ISHIBASHI, Masaki**
**Higashikagawa-shi, Kagawa 769-2712 (JP)**
- **HAMAMOTO, Hidetoshi**
**Higashikagawa-shi, Kagawa 769-2712 (JP)**

(74) Representative: **Vossius & Partner**
**Patentanwälte Rechtsanwälte mbB**
**Siebertstraße 3**
**81675 München (DE)**

(54) **ANTI-INFLAMMATORY ANALGESIC EXTERNAL PREPARATION**

(57) The present invention provides an external preparation composition comprising lactic acid salt of lidocaine consisting of lidocaine and a lactic acid ingredient and diclofenac or a salt thereof wherein the lactic acid ingredient is lactic acid and an alkali metal salt or alkaline earth metal salt of lactic acid, and an external preparation which exhibits higher transdermal absorbability of both lidocaine and diclofenac and the skin permeability suitable for clinical use, and enhances the storage stability and safety of the preparation to allow the long-term storage.

[Fig. 2]

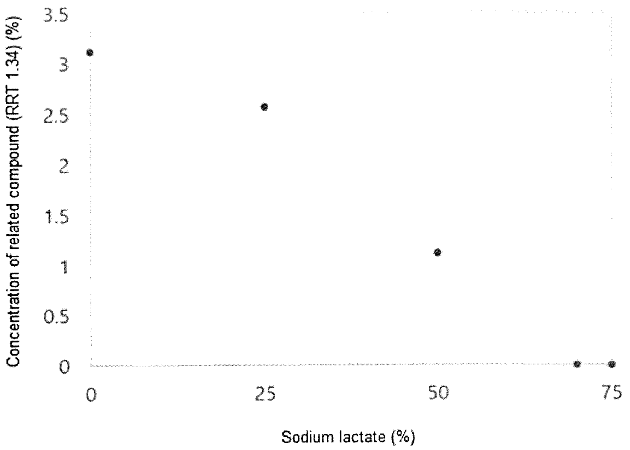


EP 3 900 713 A1

## Description

TECHNICAL FIELD

(CROSS-REFERENCES TO RELATED APPLICATIONS)

This patent application claims the benefit of Japanese Patent Application No. 2018-237691 filed on December 19, 2018. The contents of this application are hereby incorporated by this reference in its entirety.

The present invention relates to an external preparation comprising lidocaine and diclofenac or a salt thereof as an active ingredient. More specifically, the present invention relates to an external preparation composition comprising an equimolar salt of lidocaine and a lactic acid ingredient (lactic acid salt of lidocaine) and diclofenac or a salt thereof, wherein the lactic acid ingredient is lactic acid and an alkali metal salt or alkaline earth metal salt of lactic acid as well as an external preparation comprising the same.

BACKGROUND ART

Various external preparations comprising a non-steroidal antiphlogistic-analgesic agent and a local anesthetic agent are suggested (e.g., Patent Documents 1-5). Diclofenac sodium, which is one of the commonly-used non-steroidal antiphlogistic-analgesics, had low solubility in solvents, and thus was difficult to be formulated as an external preparation such as patch preparation. Even if such external preparation was prepared, the pharmaceutical effect of diclofenac was not sometimes produced because of insufficient skin permeability.

In order to enhance the skin permeability of diclofenac, some attempts to form an ionic liquid by combining diclofenac with a local anesthetic agent have been done. In Patent Documents 2-5, it has been reported that certain results such as the depression of melting point, the improvement of solubility in organic solvents and the reduction of skin irritation were achieved by the formation of ion pairs.

However, the solubility of diclofenac-lidocaine salt in solvents was still insufficient in the preparation of external preparations comprising diclofenac and lidocaine. Hence, further improvement of the preparations has been desired. It has not been reported that external preparations comprising diclofenac and lidocaine could be clinically used.

As the techniques for easily dissolving lidocaine in an organic solvent to enhance the transdermal absorbability of lidocaine, it has been known that lidocaine is reacted with an equimolar amount of lactic acid to produce lactic acid salt of lidocaine in the ionic liquid form (Patent Document 6). However, Patent Document 6 neither discloses nor suggests that lactic acid and an alkali metal salt or alkaline earth metal salt of lactic acid are used to prepare lactic acid salt of lidocaine.

PRIOR ART DOCUMENTS

PATENT DOCUMENTS

Patent Document 1: JP 2002-238699
Patent Document 2: JP 2003-335663
Patent Document 3: JP 2004-323502
Patent Document 4: JP 2005-145931
Patent Document 5: JP 2005-82512
Patent Document 6: WO 2009/060629

SUMMARY OF INVENTION

(PROBLEM TO BE SOLVED BY THE INVENTION)

An object of the present invention is to provide an external preparation comprising lidocaine and diclofenac or a salt thereof as active ingredients which exhibits higher transdermal absorbability of both active ingredients and the skin permeability suitable for clinical use, and enhances the storage stability and safety of the preparation to allow the long-term storage.

(MEANS FOR SOLVING THE PROBLEMS)

The present inventors have extensively studied external preparations comprising lidocaine and diclofenac or a salt thereof, and then have found that when an appropriate amount of diclofenac sodium is dissolved in an equimolar salt of lidocaine and lactic acid (an ionic liquid), lidocaine and diclofenac are dissolved in solution without the precipitation of crystals thereof, and thus external preparations with the skin permeability suitable for clinical use can be prepared. On the other hand, the present inventors have found that when such external preparation is stored at room temperature for 1 year or more, the related compounds of lidocaine and diclofenac are generated as impurities. Based on the findings, they tried to replace a part of lactic acid added in the preparation of lactic acid salt of lidocaine with an alkali metal salt or alkaline earth metal salt of lactic acid (e.g., sodium lactate). As a result, they have found that the generation of the related compounds of lidocaine and diclofenac are inhibited or prevented, and the skin permeability of both lidocaine and diclofenac is improved. Based upon the new findings, the present invention has been completed.

That is, the present invention provides the following embodiments.

[1] An external preparation composition comprising lactic acid salt of lidocaine consisting of lidocaine and a lactic acid ingredient, and diclofenac or a salt thereof, wherein the lactic acid ingredient is lactic acid and an alkali metal salt or alkaline earth metal salt of lactic acid.

[2] The external preparation composition according to the item [1], wherein the alkali metal salt of lactic acid is sodium lactate.

[3] The external preparation composition according to the item [1] or [2], wherein the concentration of sodium lactate in the lactic acid ingredient is 55 mol% or more.

[4] The external preparation composition according to any one of the items [1] to [3], wherein the concentration of sodium lactate in the lactic acid ingredient is 60 mol% or more.

[5] The external preparation composition according to any one of the items [1] to [4], wherein the concentration of the lactic acid salt of lidocaine is 2 to 5 moles per mole of diclofenac or a salt thereof.

[6] The external preparation composition according to any one of the items [1] to [5], wherein the amount of the lactic acid salt of lidocaine is 5 to 40% by weight.

[7] The external preparation composition according to any one of the items [1] to [6], wherein the amount of diclofenac or a salt thereof is 1 to 20% by weight.

[8] The external preparation composition according to any one of the items [1] to [7] with a total ion concentration of 0.009 mole to less than 0.057 mole per 20 g of the external preparation composition.

[9] The external preparation composition according to any one of the items [1] to [8], which further comprises an ester.

[10] The external preparation composition according to any one of the item [9], wherein the ester is diethyl sebacate, methyl laurate, diisopropyl adipate, isopropyl myristate, propylene carbonate or a mixture thereof.

[11] The external preparation composition according to any one of the items [1] to [10], which further comprises an antioxidant.

[12] The external preparation composition according to the item [11], wherein the antioxidant is dibutylhydroxytoluene (BHT), butylhydroxyanisole (BHA), propyl gallate or a mixture thereof.

[13] An external preparation comprising the external preparation composition according to any one of the items [1] to [12].

[14] The external preparation according to the item [13], which is a matrix-type patch preparation (a tape preparation).

[15] The external preparation according to the item [13] or [14] composed of a support, an adhesive layer comprising an active ingredient and a release liner.

[16] The external preparation according to the item [15], wherein the adhesive layer comprises a polymer with a dispersed solution comprising lactic acid salt of lidocaine and diclofenac or a salt thereof.

[17] A method of preparing the external preparation composition according to the item [1], which comprises: mixing lidocaine and a lactic acid ingredient to produce lactic acid salt of lidocaine which is in liquid state at ambient temperature; and
dissolving diclofenac or a salt thereof in the lactic acid salt of lidocaine.

[18] The method according to the item [17], wherein the lactic acid ingredient is lactic acid and sodium lactate.

(EFFECTS OF THE INVENTION)

The present invention can relieve various pains including both inflammatory pain and neuropathic pain with the addition of two types of active ingredients with analgesic effect that have different mechanisms of action (lidocaine and diclofenac). Also, the present invention can produce good skin permeability of lidocaine and diclofenac and inhibit the reduced adhesion to the skin when they are prepared as a tape preparation, because both lidocaine and diclofenac are dissolved in solution.

In addition, the replacement of a part of lactic acid with an alkali metal salt of lactic acid (e.g., sodium lactate) or an alkaline earth metal salt of lactic acid (e.g., calcium lactate) can inhibit or prevent the generation of impurities during long-term storage, and thus improve the storage stability and safety of a preparation to allow the long-term storage.

BRIEF DESCRIPTION OF THE DRAWINGS

Fig. 1 shows the measured result of high performance liquid chromatography (HPLC) for the preparation of Comparative Example 1 stored at room temperature for 1 year and 2 months. The peak of RRT0.40 shows an unknown compound (lidocaine-derived compound), the peak of RRT0.80 shows Diclofenac related compound A, and the peak of RRT1.34 shows an ester of diclofenac and lactic acid.

Fig. 2 is a graph representing the change in the concentration of the related compound (RRT1.34) (%) relative to the concentration of sodium lactate in the lactic acid ingredient (mol%).

Fig. 3 is a graph representing the change in the concentration of the related compound (RRT0.80) (%) relative to the total concentration of ions (mol)/20 g in the preparations of Examples 3 and 5-9.

Fig. 4 is a graph representing the skin permeation amounts of lidocaine and diclofenac after 12 hr ($\mu g/cm^2$) in the preparation of Example 8, the preparation of Comparative Example 1, Lidoderm preparation and Flector preparation in the *in vitro* skin permeation test on miniature pig.

Fig. 5 is a graph representing the change in the skin permeability of diclofenac sodium and lidocaine when the ratio of sodium lactate in the lactic acid ingredient is changed in the *in vitro* skin permeation test on miniature pig. (A) is a graph representing the skin permeability of diclofenac sodium in the preparations with a concentration of sodium lactate in the lactic acid ingredient of 50%, 55%, 60%, 65%, 70% or 75% (the relative value of the skin permeation amount of diclofenac sodium per unit area ($\mu g/cm^2$) in each preparation when the skin permeation amount thereof per unit area in the preparation of Comparative Example 1 is defined as 1), and (B) is a graph representing the skin permeability of lidocaine in the preparations with a concentration of sodium lactate in the lactic acid ingredient of 50%, 55%, 60%, 65%, 70% or 75%.

DESCRIPTION OF EMBODIMENTS

Hereinafter, the embodiments of the present invention are explained in detail. As used herein, a numerical value accompanied with the term "about" is intended to include any value within the range of ± 2% of that value. The numerical range defined by both ends covers all values between the both ends as well as the values at the both ends. For example, "about 5%" means "5% ± 2%". However, the numerical value is never 0% or less.

As used herein, the term "lidocaine" means a compound of the following formula:

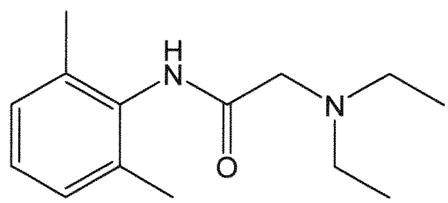

which is in solid state at ambient temperature.

In the present invention, lidocaine may form an ion pair with a lactic acid ingredient to be contained as lactic acid salt of lidocaine in a preparation.

As used herein, the term "lactic acid ingredient" means lactic acid and an alkali metal salt or alkaline earth metal salt of lactic acid such as sodium lactate, potassium lactate and calcium lactate, and involves in the formation of lactic acid salt of lidocaine (equimolar salt) produced by forming an ion pair with an equimolar amount of lidocaine. The above alkali metal salt and alkaline earth metal salt of lactic acid also encompass the alkali metal salt of lactic acid produced by the reaction of an alkali metal hydroxide such as sodium hydroxide and lactic acid used as starting materials during the preparation process and the alkaline earth metal salt of lactic acid produced by the reaction of an alkaline earth metal hydroxide such as calcium hydroxide and lactic acid used starting materials during the preparation process.

The lactic acid ingredient of the present invention is preferably ingredients comprising lactic acid and sodium lactate, more preferably lactic acid and sodium lactate or potassium lactate, and particularly preferably lactic acid and sodium lactate. The amount of the lactic acid ingredient may appropriately be determined depending on the amount of lidocaine.

The concentration of an alkali metal salt or alkaline earth metal salt of lactic acid (mol%) is, for example, about 25% or more, preferably about 50% or more, more preferably about 55% or more, relative to the total mole concentration of the lactic acid ingredient. The concentration of an alkali metal salt or alkaline earth metal salt of lactic acid (mol%) may be about 60% or more or about 65% or more, relative to the total mole concentration of the lactic acid ingredient.

The concentration of an alkali metal salt or alkaline earth metal salt of lactic acid relative to the total mole concentration of the lactic acid ingredient (mol%) is, for example, about 99% or less, about 95% or less, about 90% or less, about 85% or less, or about 80% or less.

In addition, the concentration of an alkali metal salt of lactic acid (e.g., sodium lactate) or an alkaline earth. metal salt of lactic acid (e.g., calcium lactate) (mol%) may be about 25%, about 30%, about 40%, about 45%, about 50%, about 55%, about 60%, about 65%, about 70%, about 75%, about 80%, about 85%, about 90%, about 95% or about 99%, relative to the total mole concentration of the lactic acid ingredient.

As used herein, the term "lactic acid salt of lidocaine" means an ionic liquid (an ambient temperature molten salt) produced by forming an ion pair of lidocaine and a lactic acid ingredient in equimolar amounts, which is in a viscous liquid state at ambient temperature.

The amount of lactic acid salt of lidocaine in the external preparation composition is, for example, about 5 to 40% by weight, preferably about 10 to 35% by weight, and more preferably about 20 to 30% by weight. Also, the amount of lactic acid salt of lidocaine may be about 5% by weight, about 10% by weight, about 15% by weight, about 20% by weight, about 25% by weight, about 30% by weight, about 35% by weight or about 40% by weight.

The lactic acid salt of lidocaine may be prepared as an equimolar salt of lidocaine and lactic acid by mixing lidocaine and lactic acid in the presence or absence of solvent and heating (for example, at 80°C). Also, the lactic acid salt of lidocaine may be prepared by mixing lidocaine and lactic acid at room temperature.

In the present invention, the lactic acid salt of lidocaine may be prepared as an equimolar salt produced by the reaction of a part of lidocaine and a part of lactic acid. Hence, unreacted lidocaine and lactic acid may be contained in the preparation.

The external preparation composition of the present invention may comprise, for example, unreacted lidocaine, lactic acid and/or sodium lactate.

As used herein, the term "diclofenac" means a compound of the following formula:

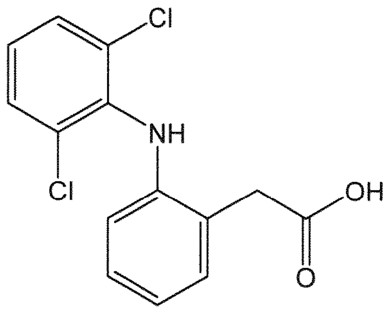

Diclofenac is usually used as an alkali metal salt such as sodium salt and potassium salt, or an organic amine salt such as epolamine salt, but is not limited thereto.

In the present invention, the salt of diclofenac is not particularly limited as long as it is a pharmaceutically acceptable salt such as a metal salt and a salt with free acid or free base. Examples of the salt of diclofenac include alkali metal salts such as sodium salt and potassium salt; alkaline earth metal salts such as calcium salt and magnesium salt; ammonium salt; organic amine salts such as dimethylamine salt, diethylamine salt, trimethylamine salt, triethylamine salt and epolamine salt, but are not limited thereto. Diclofenac or a salt thereof of the present invention is preferably diclofenac sodium or diclofenac potassium.

The amount of diclofenac or a salt thereof in the external preparation composition is, for example, about 1 to 20% by weight, preferably about 2 to 20% by weight, more preferably about 5 to 10% by weight. When diclofenac or a salt thereof is diclofenac sodium, the amount thereof may be, for example, about 1% by weight, about 2% by weight, about 5% by weight, about 10% by weight, about 15% by weight or about 20% by weight.

The external preparation composition of the present invention comprises, for example, 2 to 5 moles, 2 to 4 moles or 2.5 to 3.5 moles of lactic acid salt of lidocaine per mole of diclofenac or a salt thereof. In the external preparation composition of the present invention, the amount of lactic acid salt of lidocaine is preferably 2 to 5 moles per mole of diclofenac or a salt thereof. When lidocaine and diclofenac or a salt thereof are contained with the above range, each transdermal absorbability of lidocaine and diclofenac is improved.

In the present invention, diclofenac or a salt thereof may be in the state dissolved in lactic acid salt of lidocaine. Since the salt of diclofenac and lidocaine is normally a poorly-soluble salt, and thus it is difficult to dissolve diclofenac

in a preparation. Since the lactic acid salt of lidocaine is an ionic liquid, diclofenac is dissolved as a conjugated ionic liquid which is in the state that diclofenac is dissolved in the ionic liquid. Thus, in the ionic liquid of the present invention, the salt of diclofenac and lidocaine is contained in the dissolved state. The conjugated ionic liquid is composed of three or more types of ion sources and is an ionic liquid which is in liquid state at ambient temperature or 100°C or less even when each of the ion sources is in the salt form. As a result, the crystals of lidocaine and diclofenac is less likely to be formed in preparations. Thus, it is possible to provide preparations without the precipitation of the crystals of lidocaine, diclofenac and a salt thereof.

The external preparation composition of the present invention may be prepared by, for example, mixing lidocaine, a lactic acid ingredient and diclofenac or a salt thereof at room temperature or with heating (for example, at about 80°C). For example, the external preparation composition of the present invention may be prepared by a method comprising mixing lidocaine and a lactic acid ingredient (e.g., lactic acid and sodium lactate) to produce lactic acid salt of lidocaine which is in liquid state at ambient temperature; and dissolving diclofenac or a salt thereof in the produced lactic acid salt of lidocaine.

The external preparation composition of the present invention may comprise other agent (s) such as an organic solvent, a surfactant and an antioxidant, as appropriate.

Examples of the organic solvent include an alcohol, an ester, a fatty acid and an amine, but are not limited thereto. The organic solvent may be used alone, and two or more of the organic solvents may be used in combination.

Examples of the alcohol include monovalent alcohol such as lauryl alcohol, myristyl alcohol, oleyl alcohol, isostearyl alcohol and cetyl alcohol; divalent alcohol such as propylene glycol, butylene glycol, dipropylene glycol, diisobutylene glycol, polyethylene glycol and hexylene glycol; trivalent alcohol such as glycerin and hexanetriol, but are not limited thereto. The alcohol may be used alone, and two or more of the alcohols may be used in combination.

Examples of the ester include diethyl sebacate, methyl laurate, diisopropyl adipate, isopropyl myristate and propylene carbonate, but are not limited thereto. The ester may be used alone, and two or more of the esters may be used in combination.

Examples of the fatty acid include saturated or unsaturated fatty acid such as levulinic acid, capric acid, lauric acid, myristic acid, palmitic acid, stearic acid, isostearic acid and oleic acid, but are not limited threreto. The fatty acid may be used alone, and two or more of the fatty acids may be used in combination.

Examples of the amine include monoethanolamine, diethanolamine, diisopropanolamine, triethanolamine, triisopropanolamine, ethylenediamine and trishydroxymethylaminomethane, but are not limited thereto. The amine may be used alone, and two or more of the amines may be used in combination.

The amount of the organic solvent in the external preparation composition is, for example, 1 to 30% by weight, preferably 1 to 20% by weight, more preferably 1 to 10% by weight. Also, the organic solvent may comprise water in an amount of less than 1.0% by weight.

For the external preparation composition of the present invention prepared as a matrix-type patch preparation (a tape preparation), when the amount of the organic solvent exceeds 30% by weight, the adhesive layer in the patch preparation may be soften. As a result, it is sometimes difficult to prepare such preparation.

Examples of the surfactant include non-ionic surfactant such as monoglyceride stearate and polyoxyethylene castor oil; anionic surfactant such as sodium lauryl sulfate and potassium lauryl sulfate; and cationic surfactant such as benzalkonium chloride and stearyltrimethylammonium chloride. The surfactant may be used alone, and two or more of the surfactants may be used in combination.

The amount of the surfactant in the external preparation composition is, for example, 0.01 to 2% by weight, preferably 0.01 to 1% by weight.

Examples of the antioxidant include dibutylhydroxytoluene (BHT), butylhydroxyanisole (BHA), propyl gallate, ascorbic acid, sodium sulfite and sodium pyrosulfite, but are not limited thereto. The antioxidant may be used alone, and two or more of the antioxidants may be used in combination.

The amount of the antioxidant in the external preparation composition is, for example, 0.01 to 5% by weight, preferably 0.01 to 2% by weight, more preferably 0.01 to 1% by weight.

The external preparation composition of the present invention may further comprise various types of additives used for preparing conventional external preparations, for example, pH adjuster. The pH adjuster may be any compound as long as it is an acid, a base or a salt thereof commonly used for adjusting the pH of a preparation in the pharmaceutical field. Examples thereof include hydrochloric acid, sulfuric acid, phosphoric acid, citric acid, gluconic acid, succinic acid, acetic acid, methanesulfonic acid, edetic acid, ammonia solution, monoethanolamine, diethanolamine, triethanolamine, diisopropanolamine, triisopropanolamine, meglumine, trometamol, glycine, potassium hydroxide, calcium hydroxide, sodium hydroxide, magnesium hydroxide, sodium citrate, sodium acetate, sodium hydrogen carbonate, potassium hydrogen carbonate and sodium carbonate.

As used herein, the term "total ion concentration" means the total amount of positive and negative ions separated from each ingredient in the external preparation composition, and the unit thereof is expressed as "mol". The concentration of ions separated from each ingredient is calculated by multiplying the concentration of each ingredient by the number

of the generated ions per molecule of each ingredient. For example, sodium lactate is separated into two ions: lactate ion and sodium ion, and thus twice the concentration of sodium lactate corresponds to the ion concentration of sodium lactate. When the external preparation composition of the present invention comprises lidocaine, lactic acid, sodium lactate and diclofenac sodium, the total ion concentration of the composition may be calculated according to the following formula.

Total ion concentration = (Lidocaine (mol)) + (Lactic acid (mol)) + (Sodium lactate (mol)) x 2 + (Diclofenac sodium (mol)) x 2

In the present invention, the total ion concentration of the external preparation composition is preferably about 0.008 mol to about 0.060 mol, more preferably about 0.009 mol to about 0.057 mol, and furthermore preferably about 0.009 mol to about 0.055 mol per 20 g of the external preparation composition. For example, the total ion concentration of the external preparation composition may be about 0.009 mol to less than about 0.057 mol per 20 g of the external preparation composition.

Also, the total ion concentration of the external preparation composition may be about 0.008 mol, about 0.009 mol, about 0.010 mol, about 0.015 mol, about 0.020 mol, about 0.025 mol, about 0.030 mol, about 0.035 mol, about 0.040 mol, about 0.045 mol, about 0.050 mol, about 0.055 mol, about 0.056 mol, about 0.057 mol, about 0.058 mol, about 0.059 mol or about 0.060 mol per 20 g of the external preparation composition.

The external preparation composition of the present invention may be formulated into a dosage form capable of directly administering active ingredients in a preparation onto the local surface of the skin, and may be used as any preparation such as patch preparation, cream agent, ointment, cataplasm, aerosol and external powder. The external preparation composition is preferably formulated as tape preparation. As used herein, the external preparation means a preparation formulated from the external preparation composition.

The external preparation composition of the present invention may be formulated as a patch preparation with the three-layer structure composed of a support, an adhesive layer comprising active ingredients and a release liner. For example, the patch preparation may form the structure in which the adhesive layer is laminated on the one side of the support and the release liner is laminated on the opposite side of the adhesive layer laminated on the support. An example of the patch preparations formulated from the external preparation composition of the present invention includes a matrix-type patch preparation (a tape preparation).

As the support in the patch preparation of the present invention, a drug-impermeable and stretchable or unstretchable support may be used. The support is not particularly limited thereto as long as it is usually used in the pharmaceutical field. Examples thereof include polyethylene, polypropylene, polybutadiene, ethylene-vinyl acetate copolymer, polyvinyl chloride, polyester (such as polyethylene terephthalate), film or sheet of synthetic resin such as nylon and polyurethan or laminated product thereof, porous material, foam, film with deposited aluminum, paper, woven cloth and non-woven cloth.

The external preparation of the present invention can be prepared as a matrix-type patch preparation by dispersing the external preparation composition of the present invention in an adhesive layer comprising an appropriate polymer (elastomer).

The polymer of the present invention includes an acrylic polymer, a rubber polymer, a silicone polymer, and a vinyl ether-based polymer, but is not limited thereto. The polymer may be used alone, and two or more of the polymers may be used in combination.

Examples of the acrylic polymer include acrylic acid-acrylic acid octyl ester copolymer, 2-ethylhexyl acrylate-vinylpyrrolidone copolymer, 2-ethylexyl acrylate-N-vinyl-2-pyrrolidone-dimethacrylic acid-1,6-hexaneglycol copolymer, acrylate-vinyl acetate copolymer and 2-ethylhexyl acrylate-2-hydroxyethyl acrylate-vinyl acetate copolymer, but are not limited thereto.

Examples of the rubber polymer include synthetic rubbers such as styrene-isoprene-styrene block copolymer (hereinafter, also referred to as "SIS"), styrene-butadienestyrene block copolymer, styrene-ethylene-butadiene rubber-styrene block copolymer, styrene-butadiene rubber, polyisoprene, polyisobutylene and polybutene; and natural rubber, but are not limited thereto.

Examples of the silicone polymer include silicone rubber, dimethylpolysiloxane, diphenylpolysiloxane, but are not limited thereto.

The adhesive layer may further comprise other additive(s) such as a tackifier and a softener.

Examples of the tackifier include rosin ester, hydrogenated rosin ester, maleic modified rosin, cycloaliphatic saturated hydrocarbon resin, terpene resin and polyolefin resin, but are not limited thereto.

Examples of the softener include naphthenic base process oil, vegetable oils such as camellia oil and castor oil, liquid rubbers such as liquid polybutene and liquid isoprene rubber, liquid paraffin, but are not limited thereto.
When the external preparation of the present invention is prepared as a matrix-type patch preparation, the amount of the lactic acid salt of lidocaine may be in the range of about 5 to 40%, about 10 to 35%, about 20 to 30% or about 25 to 30% relative to the weight of the adhesive layer.
When the external preparation of the present invention is prepared as matrix-type patch preparation, the solvent method may be used as the preparation method. Examples of the solvent used in the solvent method include toluene, ethyl acetate, heptane and a mixture thereof, but are not limited thereto. The solvent is preferably toluene.
The release liner in the patch preparation of the present invention can protect the adhesive layer until the patch preparation is applied to the skin. As the packaging material for packaging the patch preparation of the present invention, aluminum laminated film can be used. In the innermost layer of the aluminum laminated film, a material such as polyacrylonitrile, polyethylene terephthalate and polyolefin can be used.
The amount of the external preparation of the present invention to be used varies with various factors such as the symptom and age of patients. In general, the external preparation of the present invention is preferably administered to adults once to several times a day. More preferably, the external preparation of the present invention is administered once to twice a day, but the number of administration may be increased 5 the symptom of patients.
The external preparation of the present invention can be used in the treatment of various types of pains including inflammatory pain and neuropathic pain. For example, the external preparation of the present invention is effective for chronic pain such as rheumatism.

EXAMPLES

Hereinafter, the present invention is described more specifically with reference to Examples, Comparative Examples and Test Examples. However, the present invention is not intended to be limited to them by any means.
For each ingredient in the table below, the following products were used.

- Lidocaine (Japanese Pharmacopoeia (JP) Lidocaine)
- Lactic acid (JP Lactic acid 90%)
- Sodium lactate (Sodium lactate solution about 70%)
- Diclofenac sodium (Japanese Pharmacopoeia (JP) Diclofenac sodium)
- IPM (isopropyl myristate)
- Liquid paraffin (Viscosity, SUS(37.8°C) 340-410)
- SIS (SIS-5002, manufactured by JSR Corporation)
- Terpene resin (PX1150N, manufactured by Yasuhara Chemical Co., Ltd.)
- BHT (2,6-di-t-butyl-4-methylphenol)
- BHA (3(2)-t-butyl-4-hydroxyanisole)

<u>Examples 1-4</u>

Each ingredient was weighed in the amount shown in Table 1 below to prepare the preparations of Examples 1-4. Specifically, according to the solvent method, styrene-isoprene-styrene block copolymer (SIS) and terpene resin were dissolved in toluene, 1) liquid paraffin, 2) lactic acid, sodium lactate and isopropyl myristate and then 3) lidocaine were added to the solution to generate lactic acid salt of lidocaine which is in liquid state at ambient temperature produced by forming an ion pair of lidocaine and the lactic acid ingredient, and then diclofenac sodium was added thereto and mixed. The mixture was then coated onto a silicone-treated PET film and dried to remove toluene to provide a plaster, and the resulting plaster was laminated onto a support and cut to the 7 cm x 10 cm size to prepare each preparation.

[Table 1]

| | Example 1 | Example 2 | Example 3 | Example 4 |
|---|---|---|---|---|
| Ingredient Name | Amount (g) (Formulation ratio (%)) | | | |
| Lidocaine | 4.0 g (20.0%) | 4.0 g (20.0%) | 4.0 g (20.0%) | 4.0 g (20.0%) |
| mol | 0.017 | 0.017 | 0.017 | 0.0017 |
| Lactic acid | 1.29 g (6.46%) | 0.86 g (4.31%) | 0.52 g (2.58%) | 0.43 g (2.15%) |
| mol | 0.013 | 0.009 | 0.005 | 0.004 |
| Sodium lactate | 0.65 g (3.27%) | 1.31 g (6.54%) | 1.83 g (9.16%) | 1.96 g (9.81%) |
| mol | 0.004 | 0.009 | 0.012 | 0.013 |
| Diclofenac sodium | 1.6 g (8.0%) | 1.6 g (8.0%) | 1.6 g (8.0%) | 1.6 g (8.0%) |
| mol | 0.005 | 0.005 | 0.005 | 0.005 |
| IPM | 1.6 g (8.0%) | 1.6 g (8.0%) | 1.6 g (8.0%) | 1.6 g (8.0%) |
| Liquid paraffin | 2.35 g (11.8%) | 2.13 g (10.7%) | 1.95 g (9.8%) | 1.91 g (9.5%) |
| SIS | 2.1 g (10.5%) | 2.1 g (10.5%) | 2.1 g (10.5%) | 2.1 g (10.5%) |
| Terpene resin | 6.4 g (32.0%) | 6.4 g (32.0%) | 6.4 g (32.0%) | 6.4 g (32.0%) |
| Total | 20.0 g (100.0%) | 20.0 g (100.0%) | 20.0 g (100.0%) | 20.0 g (100.0%) |
| | | | | |
| Sodium lactate /Lactic acid ingredient *1 | 25% | 50% | 70% | 75% |
| Total ion concentration*2 | 0.049 mol | 0.054 mol | 0.057 mol | 0.058 mol |

*1

*1 Sodium lactate/Lactic acid ingredient (mol%) = Sodium lactate (mol)/Lactic acid ingredient (mol)

*2

*2 Total ion concentration = (Lidocaine (mol)) + (Lactic acid (mol)) + (Sodium lactate (mol)) x 2 + (Diclofenac sodium (mol)) x 2

Examples 5-9

Each ingredient was weighed in the amount shown in Table 2 below to prepare the preparations of Examples 5-9. Specifically, according to the solvent method, styrene-isoprene-styrene block copolymer (SIS) and terpene resin were dissolved in toluene, 1) liquid paraffin, 2) dibutylhydroxyltoluene (BHT) and butylhydroxyanisole (BHA), 3) lactic acid, sodium lactate and isopropyl myristate and then 4) lidocaine were added to the solution to generate lactic acid salt of lidocaine which is in liquid state at ambient temperature produced by forming an ion pair of lidocaine and the lactic acid ingredient, and then diclofenac sodium was added thereto and mixed. The mixture was then coated onto a silicone-treated PET film and dried to remove toluene to provide a plaster, and the resulting plaster was laminated onto a support and cut to the 7 cm x 10 cm size to prepare a preparation.

[Table 2]

| Ingredient Name | Example 5 | Example 6 | Example 7 | Example 8 | Example 9 |
|---|---|---|---|---|---|
| Amount (g) (Formulation ratio (%)) | | | | | |
| Lidocaine | 3.3 g (16.5%) | 3.0 g (15.0%) | 3.4 g (17.0%) | 3.4 g (17.0%) | 4.0 g (20.0%) |
| mol | 0.014 | 0.013 | 0.015 | 0.015 | 0.017 |
| Lactic acid | 0.38 g (1.90%) | 0.39 g (1.94%) | 0.44 g (2.20%) | 0.44 g (2.20%) | 0.52 g (2.60%) |
| mol | 0.004 | 0.004 | 0.004 | 0.004 | 0.005 |
| Sodium lactate | 1.58 g (7.92%) | 1.37 g (6.87%) | 1.56 g (7.80%) | 1.56 g (7.80%) | 1.83 g (9.20%) |
| mol | 0.010 | 0.009 | 0.010 | 0.010 | 0.012 |
| Diclofenac sodium | 1.6 g (8.0%) | 1.6 g (8.0%) | 1.6 g (8.0%) | 1.6 g (8.0%) | 1.6 g (8.0%) |
| mol | 0.005 | 0.005 | 0.005 | 0.005 | 0.005 |
| IPM | 1.6 g (8.0%) | 1.6 g (8.0%) | 1.6 g (8.0%) | 1.6 g (8.0%) | 1.6 g (8.0%) |
| BHT | 0.04 g (0.2%) | 0.04 g (0.2%) | 0.04 g (0.2%) | 0.04 g (0.2%) | 0.04 g (0.2%) |
| BHA | 0.02 g (0.1%) | 0.02 g (0.1%) | 0.02 g (0.1%) | 0.02 g (0.1%) | 0.02 g (0.1%) |
| Liquid paraffin | 2.97 g (14.9%) | 2.38 g (11.9%) | 2.84 g (14.2%) | 1.34 g (6.7%) | 1.19 g (5.96%) |
| SIS | 2.1 g (10.5%) | 2.8 g (14.0%) | 2.1 g (10.5%) | 3.2 g (16.0%) | 2.4 g (12.0%) |
| Terpene resin | 6.4 g (32.0%) | 6.8 g (34.0%) | 6.4 g (32.0%) | 6.8 g (34.0%) | 6.8 g (34.0%) |
| Total | 20.0 g (100.0%) | 20.0 g (100.0%) | 20.0 g (100.0%) | 20.0 g (100.0%) | 20.0 g (100.0%) |
| | | | | | |
| Sodium lactate/ Lacitic acid ingredient | 73% | 70% | 70% | 70% | 70% |
| Total ion concentration | 0.049 mol | 0.045 mol | 0.050 mol | 0.050 mol | 0.057 mol |

Comparative Example 1

Each ingredient was weighed in the amount shown in Table 3 below to prepare the preparation of Comparative Example 1. Specifically, according to the solvent method, styrene-isoprene-styrene block copolymer (SIS) and terpene resin were dissolved in toluene, 1) liquid paraffin, 2) lactic acid and isopropyl myristate and then 3) lidocaine were added to the solution to generate lactic acid salt of lidocaine which is in liquid state at ambient temperature produced by forming an ion pair of lidocaine and the lactic acid ingredient, and then diclofenac sodium was added thereto and mixed. The mixture was then coated onto a silicone-treated PET film and dried to remove toluene to provide a plaster, and the resulting plaster was laminated onto a support and cut to the 7 cm x 10 cm size to prepare a preparation.

[Table 3]

| | Comparative Example 1 |
|---|---|
| Ingredient Name | Amount (g) (Formulation ratio (%)) |
| Lidocaine | 8.0 g (20%) |
| Lactic acid | 3.44 g (8.6%) |
| Diclofenac sodium | 3.2 g (8.0%) |
| IPM | 3.2 g (8.0%) |
| Liquid paraffin | 5.16 g (12.9%) |
| SIS | 4.2 g (10.5%) |
| Terpene resin | 12.8 g (32%) |
| Total | 40.0 g (100.0%) |
| | |
| Total ion concentration | 0.045 mol |

Test Example 1: Confirmation test of related compounds

According to the following procedure, the confirmation test of the related compounds of lidocaine and diclofenac was performed for the preparation of Comparative Example 1 stored at room temperature for 1 year and 2 months.

(Preparation of sample solution)

The preparation was cut into 8 portions, each liner thereof was removed and mixed with 30 mL of tetrahydrofuran (including a stabilizing agent), and the mixture was irradiated with ultrasonic waves for 10 minutes. About 120 mL of methanol was added thereto and mixed with shaking well, and then methanol was added to adjust the solution to 200 mL. The solution was filtered with a membrane filter with a pore size of 0.45 μm or less, the first 2 mL of filtrate was removed, and the next 5 mL of filtrate was measured and adjusted to 25 mL with the addition of methanol. The solution was then filtered with a membrane filter with a pore size of 0.45 μm or less, the first 2 mL of filtrate was removed, and the filtrate thus obtained was used as sample solution.

(Preparation of standard solution)

About 16 mg of quantitative diclofenac sodium was weighed and dissolved in methanol to adjust the solution to 100 mL. 2.5 mL of the solution was measured and methanol was added to adjust the solution to 50 mL. In addition, 2.5 mL of the solution was measured and methanol was added to adjust the solution to 50 mL. The solution thus obtained was used as standard solution.

(Measurement by HPLC)

Each 10 mL of the prepared sample solution and standard solution was taken and measured by high performance liquid chromatography (HPLC) under the following conditions.

<HPLC conditions>

Detector: UV detector 210 nm
Column: Stainless steel tube with an inner diameter of 4.6 mm and a length of 15 cm, filled with 5 μm octadecylsilylated silica gel for liquid chromatography
Column temperature: Constant temperature of about 40°C
Mobile phase: Solution prepared by dissolving 3.456 g of sodium lauryl sulfate in 1200 mL of 0.02 mol/L phosphate buffer (pH 3.0) and adjusting to 2000 mL with the addition of acetonitrile
Flow rate: The retention time of lidocaine is adjusted to about 13 minutes (about 1.000 mL/min)

(Calculation of amount of related compounds)

The peak area of each ingredient was measured by the automatic integration and the amount of each related compound relative to diclofenac (%) was calculated according to the following formula.

$$\text{Amount of each related compound}(\%) = W_S \times (A_T/A_S) \times (1/40) \times (1/C) \times 100$$

$W_s$: Weighed amount of quantitative diclofenac sodium (mg)
$A_T$: Peak area of each related compound obtained from sample solution
$A_s$: Peak area of diclofenac obtained from standard solution 1/40: Dilution coefficient
C: Displayed amount of diclofenac sodium per sheet of this product

The test result is shown in Fig. 1. As shown in Fig. 1, the generation of 3 types of related compounds was confirmed (RRT0.40, RRT0.80 and RRT1.34). RRT means the relative retention time for diclofenac.

Also, it was shown that the related compound (RRT0.40) was an unknown compound (lidocaine-derived compound), the related compound (RRT0.80) was Diclofenac related compound A, and the related compound (RRT1.34) was an ester of diclofenac and lactic acid.

Test Example 2: Measurement of total amount of related compounds

The preparations of Examples 1-9 and Comparative Example 1 were stored under the conditions of 80°C x 2 days, and then the total amount of each related compound was measured. The storage under the conditions of 80°C x 2 days corresponds to the storage at room temperature for 670 days.

The guideline: "Impurities in New Drug Products" published by the International Council for Harmonisation of Technical Requirements for Pharmaceuticals for Human Use describes the baseline on the degradation products (impurities) observed during stability test of a new drug product. The preparation is determined to show excellent stability based on the guideline when the total amount of the degradation products (related compounds) is less than 0.2% of diclofenac sodium.

The relation between the concentration of the related compound (RRT1.34) in the preparations of Examples 1-4 and Comparative Example 1 as well as the concentration of sodium lactate in the lactic acid ingredient (mol%) is shown in Table 4, and the total ion concentration and the total amount of each related compound in the preparations of Examples 3 and 5-9 are shown in Table 5. Also, a graph representing the change in the concentration of the related compound (RRT1.34) (%) relative to the concentration of sodium lactate in the lactic acid ingredient (mol%) is shown in Fig. 2, and a graph representing the change in the concentration of the related compound (RRT0.80) (%) relative to the total ion concentration of each preparation of Examples 3 and 5-9 (mol/20 g of the external preparation) is shown in Fig. 3.

[Table 4]

| | Sodium lactate/Lactic acid ingredient (mol%) | Concentration of related compound (RRT1.34) (%) |
|---|---|---|
| Example 1 | 25 | 2.58 |
| Example 2 | 50 | 1.12 |
| Example 3 | 70 | 0.00 |
| Example 4 | 75 | 0.00 |
| Comparative Example 1 | 0 | 3.12 |

It was shown that the preparations which a concentration of sodium lactate relative to the total lactic acid ingredients of 70 mol% or more could completely inhibit the generation of the related compound (RRT1.34) (Table 4 and Fig. 2).

[Table 5]

| | Total ion concentration (mol) | Sodium lactate /Lactic acid ingredient (mol%) | Concentration of related compound (%) | | |
|---|---|---|---|---|---|
| | | | RRT0.40 | RRT0.80 | RRT1.34 |
| Example 5 | 0.049 | 73 | 0.03 | 0.48 | 0.00 |
| Example 6 | 0.045 | 70 | 0.00 | 0.54 | 0.00 |
| Example 7 | 0.050 | 70 | 0.03 | 0.45 | 0.00 |
| Example 8 | 0.050 | 70 | 0.04 | 0.53 | 0.00 |
| Example 3 | 0.057 | 70 | 0.16 | 0.72 | 0.00 |
| Example 9 | 0.057 | 70 | 0.12 | 0.90 | 0.05 |

It was shown that the external preparations with a total ion concentration of less than about 0.057 mole could more effectively inhibit the generation of the related compound (RRT0.80) (Table 5 and Fig. 3).

Test Example 3: *in vitro* skin permeability test on miniature pig (1)

According to the following procedure, the skin permeation amounts of the active ingredient(s) in the preparation of Example 8, the preparation of Comparative Example 1, the commercially available patch preparation comprising lidocaine (Lidoderm®) and the commercially available patch preparation comprising diclofenac (Flector®) were measured.

A Franz cell was set and was filled with saline. The Franz cell was warmed at around 32°C. A disc with a φ15 mm hole in a φ24 mm membrane filter was attached on the back side of the thawed skin of a miniature pig, the skin was punched with a φ24 mm punch, and the skin was set in the Franz cell. The excess water around the Franz cell and on the upper surface of the skin was wiped off. The skin was acclimated to the environment for about 20 minutes and then was removed. Each preparation punched to ϕ12 mm was applied to the central part of the skin, and the skin was set in the Franz cell. The excess water around the Franz cell was wiped off, the filter paper punched to φ24 mm was placed on the skin, and the cap of the Franz cell was closed and fixed with a clip. The sampling of each preparation was performed 1 hr, 3 hr, 6 hr, 9 hr and 12 hr after the start of the test, and the skin permeation amounts thereof were measured by high performance liquid chromatography (HPLC) under the following conditions.

<HPLC conditions>

Detector: UV detector 210nm
Column: Stainless steel tube with an inner diameter of 4.6 mm and a length of 15 cm, filled with 5 μm octadecylsilylated silica gel for liquid chromatography
Column temperature: Constant temperature of about 40°C
Mobile phase: Solution prepared by dissolving 5.76 g of sodium lauryl sulfate in 900 mL of 0.02 mol/L phosphate buffer (pH 3.0) and adjusting to 2000 mL with the addition of acetonitrile
Flow rate: 0.700 mL/min

The skin permeation amounts of lidocaine and diclofenac in each preparation after 12 hr are shown in Table 6 and Fig. 4.

[Table 6]

| | Example 8 | Comparative Example 1 | Lidoderm | Flector |
|---|---|---|---|---|
| Skin permeation amount of lidocaine (μg/cm$^2$) | 85.20 | 43.67 | 28.82 | 0.000 |
| Skin permeation amount of diclofenac (μg/cm$^2$) | 1.822 | 1.115 | 0.000 | 0.459 |

The above results showed that the preparation of Example 8 had better skin permeability as compared to the commercially available Lidoderm® and Flector®.

In addition, it was shown that the preparation of Example 8 enhanced the skin permeability of lidocaine and

diclofenac as compared to the preparation of Comparative Example 1.

Test Example 4: *in vitro* skin permeability test on miniature pig (2)

According to a similar procedure to the preparations of Examples 1-4, preparations with similar compositions to the preparations of Examples 1-4 and the concentration of sodium lactate in the lactic acid ingredient adjusted to 50%, 55%, 60%, 65%, 70% or 75% were prepared. For the preparations with a concentration of sodium lactate in the lactic acid ingredient of 50%, 70% or 75%, the preparations of Examples 2-4 were used. For the preparations with a concentration of sodium lactate in the lactic acid ingredient of 55%, 60% or 65%, each concentration of lactic acid and sodium lactate was changed to adjust the concentration of sodium lactate/lactic acid ingredient to 55%, 60% or 65%, and the concentrations of the lactic acid ingredient and lidocaine were adjusted in equimolar amounts, and then liquid paraffin was added to obtain each preparation with a total amount of 20.0 g.

The skin permeation amounts of diclofenac sodium and lidocaine ($\mu g/cm^2$) in each prepared preparation, the preparations of Examples 2-4 and the preparation of Comparative Example 1 were measured according to the procedure of Test Example 3.

When each skin permeation amount of diclofenac sodium and lidocaine in the preparation of Comparative Example 1 is defined as 1, the relative values of the skin permeation amounts of diclofenac sodium and lidocaine in each preparation were calculated. A graph representing the calculated relative values is shown in Fig. 5.

The result showed that in the preparations with a concentration of sodium lactate in the lactic acid ingredient of 55% or more, the skin permeability of both diclofenac sodium and lidocaine was 1 or more, and the skin permeability thereof was maximized in all of the preparations when the concentration is 70%.

Industrial Applicability

The prevent invention provides an external preparation comprising lidocaine and diclofenac or a salt thereof which exhibits higher transdermal absorbability of both active ingredients and higher storage stability and safety of the preparation. In addition, the external preparation is extremely useful in the treatment of various pains including inflammatory pain and neuropathic pain.

## Claims

1. An external preparation composition comprising lactic acid salt of lidocaine consisting of lidocaine and a lactic acid ingredient, and diclofenac or a salt thereof, wherein the lactic acid ingredient is lactic acid and an alkali metal salt or alkaline earth metal salt of lactic acid.

2. The external preparation composition according to claim 1, wherein the alkali metal salt of lactic acid is sodium lactate.

3. The external preparation composition according to claim 1 or 2, wherein the concentration of sodium lactate in the lactic acid ingredient is 55 mol% or more.

4. The external preparation composition according to any one of claims 1 to 3, wherein the concentration of sodium lactate in the lactic acid ingredient is 60 mol% or more.

5. The external preparation composition according to any one of claims 1 to 4, wherein the concentration of the lactic acid salt of lidocaine is 2 to 5 moles per mole of diclofenac or a salt thereof.

6. The external preparation composition according to any one of claims 1 to 5, wherein the amount of the lactic acid salt of lidocaine is 5 to 40% by weight.

7. The external preparation composition according to any one of claims 1 to 6, wherein the amount of diclofenac or a salt thereof is 1 to 20% by weight.

8. The external preparation composition according to any one of claims 1 to 7 with a total ion concentration of 0.009 mole to less than 0.057 mole per 20 g of the external preparation composition.

9. The external preparation composition according to any one of claims 1 to 8, which further comprises an ester.

**10.** The external preparation composition according to claim 9, wherein the ester is diethyl sebacate, methyl laurate, diisopropyl adipate, isopropyl myristate, propylene carbonate or a mixture thereof.

**11.** The external preparation composition according to any one of claims 1 to 10, which further comprises an antioxidant.

**12.** The external preparation composition according to claim 11, wherein the antioxidant is dibutylhydroxytoluene (BHT), butylhydroxyanisole (BHA), propyl gallate or a mixture thereof.

**13.** An external preparation comprising the external preparation composition according to any one of claims 1 to 12.

**14.** The external preparation according to claim 13, which is a matrix-type patch preparation (a tape preparation).

**15.** The external preparation according to claim 13 or 14 composed of a support, an adhesive layer comprising an active ingredient and a release liner.

**16.** The external preparation according to claim 15, wherein the adhesive layer comprises a polymer with a dispersed solution comprising lactic acid salt of lidocaine and diclofenac or a salt thereof.

**17.** A method of preparing the external preparation composition according to claim 1, which comprises:

mixing lidocaine and a lactic acid ingredient to produce lactic acid salt of lidocaine which is in liquid state at ambient temperature; and
dissolving diclofenac or a salt thereof in the lactic acid salt of lidocaine.

**18.** The method according to claim 17, wherein the lactic acid ingredient is lactic acid and sodium lactate.

[Fig. 1]

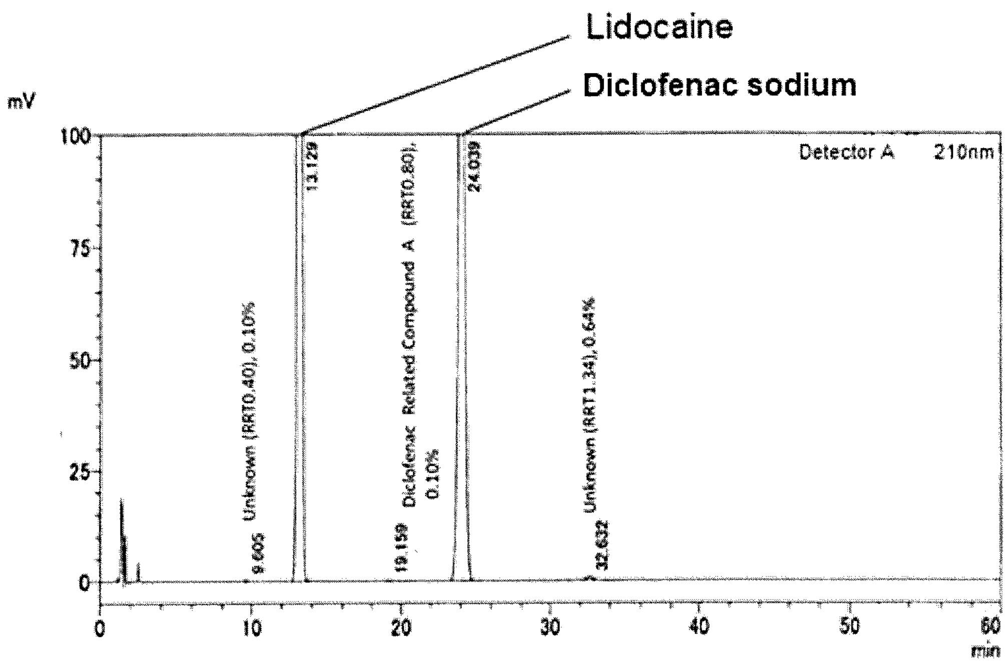
Lidocaine
Diclofenac sodium
mV
100
75
50
25
0
Detector A 210nm
13.129
24.039
Unknown (RRT0.40), 0.10%
9.605
Diclofenac Related Compound A (RRT0.80), 0.10%
19.159
Unknown (RRT1.34), 0.64%
32.632
0
10
20
30
40
50
60
min

[Fig. 2]

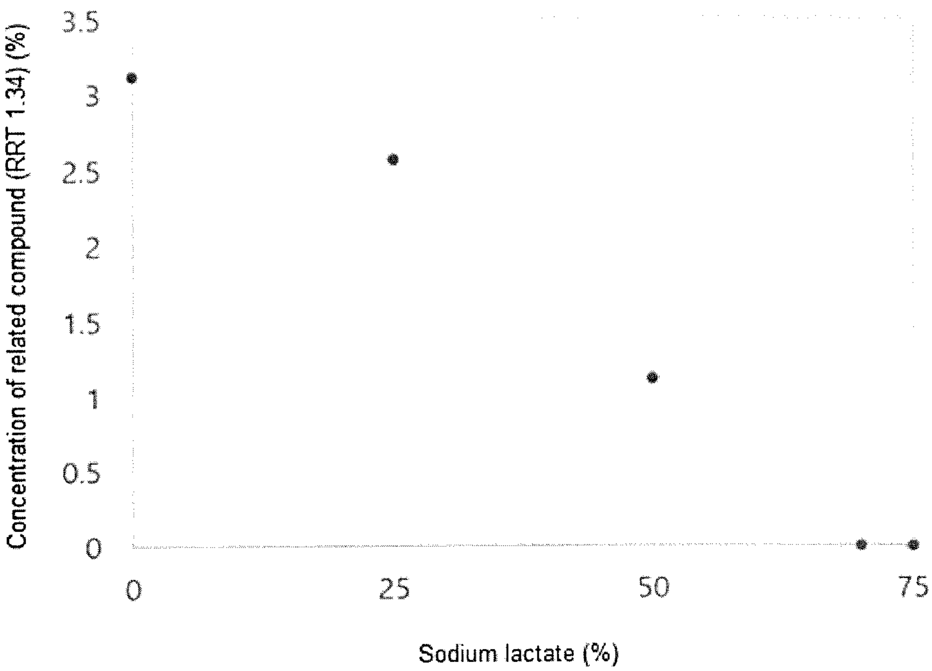
Concentration of related compound (RRT 1.34) (%)
3.5
3
2.5
2
1.5
1
0.5
0
0
25
50
75
Sodium lactate (%)

[Fig. 3]

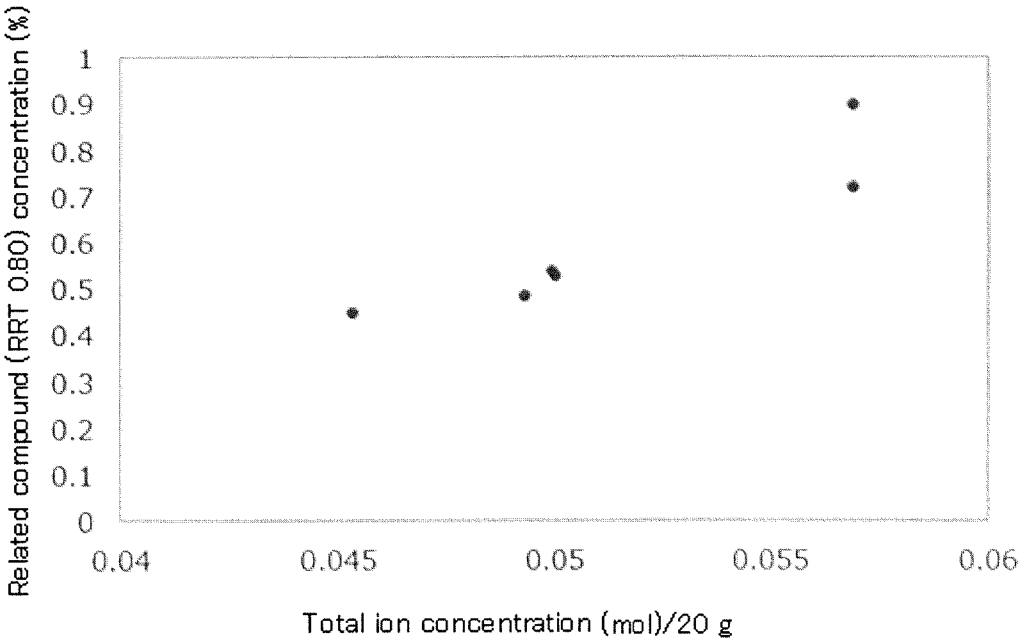
Related compound (RRT 0.80) concentration (%)
1
0.9
0.8
0.7
0.6
0.5
0.4
0.3
0.2
0.1
0
0.04
0.045
0.05
0.055
0.06
Total ion concentration (mol)/20 g

[Fig. 4]

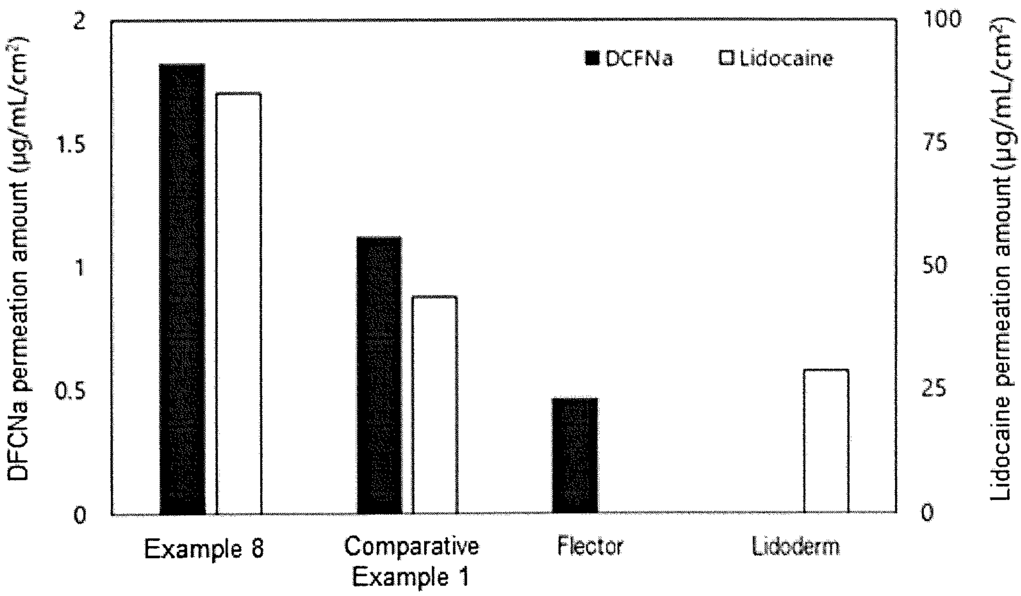
DFCNa permeation amount (μg/mL/cm²)
2
1.5
1
0.5
0
DCFNa
Lidocaine
100
75
50
25
0
Lidocaine permeation amount (μg/mL/cm²)
Example 8
Comparative Example 1
Flector
Lidoderm

[Fig. 5]

(A)

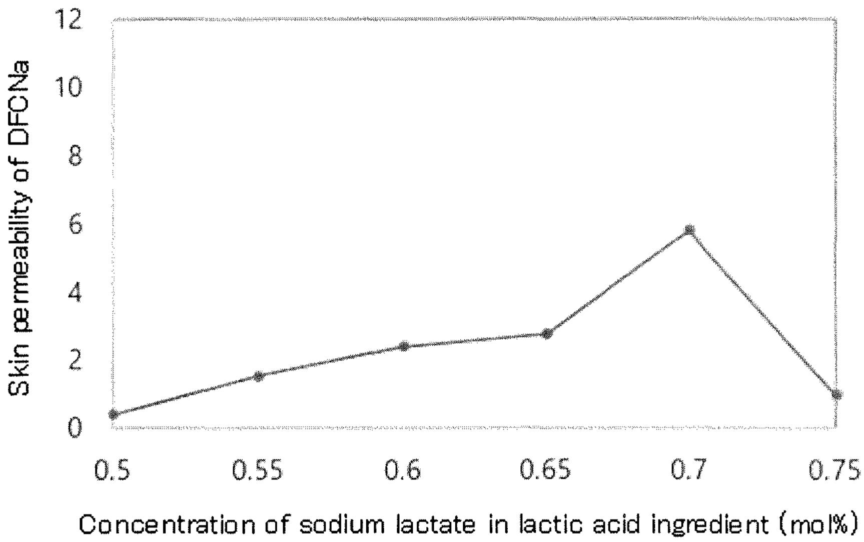
Skin permeability of DFCNa
0
2
4
6
8
10
12
0.5
0.55
0.6
0.65
0.7
0.75
Concentration of sodium lactate in lactic acid ingredient (mol%)

(B)

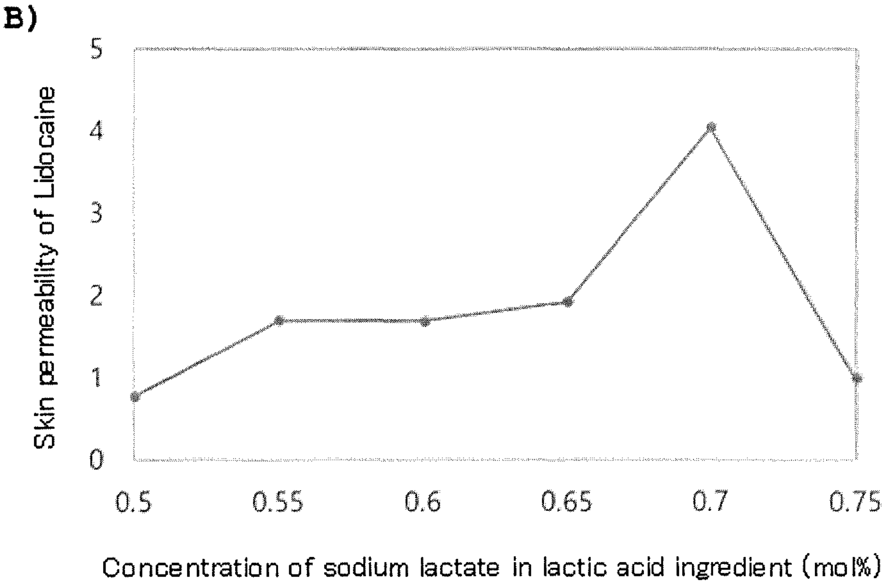
Skin permeability of Lidocaine
0
1
2
3
4
5
0.5
0.55
0.6
0.65
0.7
0.75
Concentration of sodium lactate in lactic acid ingredient (mol%)

## INTERNATIONAL SEARCH REPORT

International application No.
PCT/JP2019/049856

A. CLASSIFICATION OF SUBJECT MATTER
Int.Cl. A61K31/167(2006.01)i, A61K31/196(2006.01)i, A61P23/02(2006.01)i, A61P29/00(2006.01)i, A61P43/00(2006.01)i, A61K9/00(2006.01)i, A61K9/70(2006.01)i, A61K47/10(2006.01)i, A61K47/12(2006.01)i, A61K47/14(2006.01)i
FI: A61K31/167, A61K31/196, A61K47/12, A61K47/14, A61K47/10, A61K9/70401, A61P43/00121, A61K9/00, A61P29/00, A61P23/02
According to International Patent Classification (IPC) or to both national classification and IPC

B. FIELDS SEARCHED
Minimum documentation searched (classification system followed by classification symbols)
Int.Cl. A61K31/167, A61K31/196, A61P23/02, A61P29/00, A61P43/00, A61K9/00, A61K9/70, A61K47/10, A61K47/12, A61K47/14

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580(JDreamIII), CAplus/REGISTRY/MEDLINE/EMBASE/BIOSIS/WPIDS(STN)

C. DOCUMENTS CONSIDERED TO BE RELEVANT

| Category* | Citation of document, with indication, where appropriate, of the relevant passages | Relevant to claim No. |
|---|---|---|
| Y | JP 2005-145931 A (MEDRX CO., LTD.) 09.06.2005 (2005-06-09), particularly, claim 1, example 3, paragraphs [0023], [0025], [0026] | 1-18 |
| Y | WO 2009/060629 A1 (MEDRX CO., LTD.) 14.05.2009 (2009-05-14), particularly, paragraph [0005] | 1-18 |
| P, X | WO 2018/230687 A1 (MEDRX CO., LTD.) 20.12.2018 (2018-12-20), particularly, claims 1-8, examples 1-4 | 1-18 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 12.02.2020 | 25.02.2020 |
| Name and mailing address of the ISA/ Japan Patent Office 3-4-3, Kasumigaseki, Chiyoda-ku, Tokyo 100-8915, Japan | Authorized officer<br>Telephone No. |

Form PCT/ISA/210 (second sheet) (January 2015)

**INTERNATIONAL SEARCH REPORT**
Information on patent family members

International application No.
PCT/JP2019/049856

| | | |
|---|---|---|
| JP 2005-145931 A | 09.06.2005 | (Family: none) |
| WO 2009/060629 A1 | 14.05.2009 | US 2010/0234471 A1<br>paragraph [0007]<br>EP 2210599 A1<br>AU 2008325888 A<br>CA 2703872 A<br>CN 101861148 A<br>KR 10-2010-0086477 A<br>ES 2507567 T |
| WO 2018/230687 A1 | 20.12.2018 | (Family: none) |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2018237691 A
- JP 2002238699 A
- JP 2003335663 A
- JP 2004323502 A
- JP 2005145931 A
- JP 2005082512 A
- WO 2009060629 A

### Non-patent literature cited in the description

- Impurities in New Drug Products. International Council for Harmonisation of Technical Requirements for Pharmaceuticals for Human Use